# EUROPÄISCHE PATENTSCHRIFT

(11) **EP 1 343 483 B1**
(45) Veröffentlichungstag und Bekanntmachung des Hinweises auf die Patenterteilung: **28.05.2008**
(21) Anmeldenummer: 01985863.8
(22) Anmeldetag: 11.12.2001
(51) Int. Cl.: A61K 9/70, A61K 31/575

(54) **TRANSDERMALSYSTEM ENTHALTEND EIN HOCHPOTENTES GESTAGEN**
TRANSDERMAL SYSTEM CONTAINING A NOVEL HIGH POTENCY PROGESTAGEN
SYSTEME TRANSDERMIQUE CONTENANT UN NOUVEAU PROGESTATIF TRES PUISSANT

(30) Priorität: 21.12.2000 EP 00250449
(43) Veröffentlichungstag der Anmeldung: 17.09.2003
(73) Patentinhaber: Bayer Schering Pharma Aktiengesellschaft, 13353 Berlin (DE)
(72) Erfinder: LIPP, Ralph, 14169 Berlin (DE); GUENTHER, Clemens, 13357 Berlin (DE)
(86) Internationale Anmeldenummer: PCT/EP2001/014538
(87) Internationale Veröffentlichungsnummer: WO 2002/049622

(56) Entgegenhaltungen:
- DE-A- 19 848 303
- US-A- 5 827 842

## Beschreibung

Die Erfindung betrifft ein Transdermalsystem (TDS) von hochpotenten Gestagen(en), speziell von (21S)-21-Hydroxy-21-methyl-14,17-ethano-19-norpregna-4,9,15-trien-3,20-dion, nachfolgend als Hydroxytriendion (Fig. 1) bezeichnet. 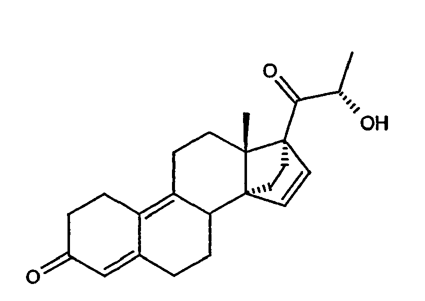 Fig. 1: Strukturformel von Hydroxytriendion

Eine besondere Form von transdermalen Formulierungen sind Transdermalsysteme.
Der Begriff "Transdermalsystem" wird im Sinne der Anmeldung in einer engen Definition für alle transdermalen Pflasterformulierungen verwendet. Üblicherweise werden Transdermalsysteme in Matrix-Transdermalsysteme und Membran-Transdermalsysteme unterteilt.

Matrix-Transdermalsysteme bestehen im einfachsten Fall aus drei parallel übereinander angeordneten Schichten, nämlich einer Rückschicht, einer Matrix und einer Abziehschicht. Letztere, die üblicherweise aus Kunststofffolie oder beschichtetem Papier besteht, wird vor der Applikation des Transdermalsystems auf die Haut entfernt. Die Matrix enthält den zu applizierenden Wirkstoff und hat üblicherweise gleichzeitig adhäsive Eigenschaften. Sollte die Matrix nicht adhäsiv genug sein, um zuverlässig an dem Hautbereich anzuhaften, auf den das Transdermalsystem appliziert werden soll, kann zwischen Matrix und Abziehschicht noch eine Kleberschicht vorgesehen werden.

Membran-Transdermalsysteme bestehen im einfachsten Fall aus vier Schichten, nämlich einer Rückschicht, einem Reservoir, einer Membran und einer Abziehschicht. Das Reservoir, das Wirk- und Hilfsstoffe enthalten kann, wird üblicherweise vollständig von Rückschicht und Membran umgeben. Die Inhaltsstoffe aus dem Reservoir können durch die Membran hindurch freigesetzt werden. Da eine Reihe von Membranen von sich aus nicht ausreichend adhäsiv sind, um an dem Hautbereich anzuhaften, auf den das Transdermalsystem appliziert werden soll, wird in der Regel zwischen Membran und Abziehschicht (zumindest im Randbereich) noch eine Kleberschicht vorgesehen.
Es ist bekannt, dass Gestagene transdermal appliziert werden können.
Die bisher in Formulierungen für transdermale Systeme eingesetzten Gestagene weisen jedoch in der Regel relativ niedrige Löslichkeiten in den verwendeten Matrizes auf, z.B. Gestoden oder Levonorgestrel ca. 1 %. Ein kristallfreies Transdermalsystem ist mit diesen Gestagenen nur herstellbar, wenn der Gehalt an Gestagen in der Matrix die Sättigungskonzentration nicht deutlich überschreitet.
Oft wird aber ein hoher Gehalt an Gestagen in der Matrix angestrebt, um ausreichende transdermale Hautflüsse zu erzielen.

Andere Gestagene besitzen zwar eine höhere Löslichkeit, wie z. B. Norethistosteron (ca. 7 %), aber ihre gestagene Potenz ist vergleichsweise gering.

DE 198 48 303 A offenbart die Kombination aus einem Gestagen, vorzugsweise Hydroxytriendion, und einem Cyclodextrin. Es wird die transdermale Applikation mittels Matrix- oder Membranpflaster vorgeschlagen.

Der Erfindung liegt daher die Aufgabe zugrunde, ein Transdermalsystem mit einem hohen Gehalt an hoch potenten Gestagenen in gelöster Form zur Verfügung zu stellen.

Erfindungsgemäß wird diese Aufgabe durch ein Matrix-Transdermalsystem mit einem Gehalt an dem hochpotenten Gestagen (21S)-21-Hydroxy-21-methyl-14,17-ethano-19-norpregna-4,9,15-trien-3,20-dion (Hydroxytriendion), wobei die Matrix Polyacrylatkleber umfasst gelöst.

Polyacrylat im Sinne des Patentes ist Oberbegriff für alle Polymere (Homo- und Copolymere), die Acrylsäure bzw. Acrylsäurederivate enthalten. Besonders bevorzugt sind Vinylacetat-Acrylat-Copolymere und Acrylat-Vinylpyrrolidon-Copolymere. Am bevorzugtesten 2-Ethylhexylacrylat-Hydroxyethylacrylat-Copolymere (Gelva^{®}) sowie Copolymere der vorgenannten Verbindungen mit weiteren Substanzen wie beispielsweise Vinylacetat und 2-Ethylhexylacrylat-N-vinyl-2-pyrrolidon (TSR^{®}-Kleber der Firma Sekisui)

Es ist ein Gehalt von 0,1 bis 20 Gew.-% Hydroxytriendion ((21S)-21-Hydroxy-21-methyl-14,17-ethano-19-norpregna-4,9,15-trien-3,20-dion), bevorzugt 1 bis 15 Gew.-%, in der Matrix vorgesehen.

In einer besonders bevorzugten Ausführungsform enthält die erfindungsgemäße transdermale Formulierung Kristallisationsinhibitoren, die als Komplexbildner geeignet sind, beispielsweise feste Lösungen mit Wirkstoffen zu bilden, die Grenzflächenlöslichkeit für den Wirkstoff erhöhen und die Neigung des Wirkstoffes zur Rekristallisation nach Entfernen eines Prozesslösungsmittels oder Senkung der Temperatur herabsetzen. Der Zusatz von Kristallisationsinhibitoren ermöglicht es, höhere Wirkstoffbeladungen der Formulierung vorzunehmen, ohne dass sich Wirkstoffkristalle bilden, die nur sehr eingeschränkt für den Stofftransport in die Haut zur Verfügung stehen. Als Kristallisationsinhibitoren sind N-Vinyllactam-Polymere wie N-Vinyl-1-Azacycloheptan-2-on-Homopolymer und N-Vinyl-piperidin-2-on-Homopolymer und insbesondere Polymere des Vinylpyrrolidons, wie Polyvidon (Kollidon^{®}), oder Co-Polymere des Vinylpyrrolidons mit Vinylacetat (Copovidone) geeignet. Besonders bevorzugt ist ein Copovidon aus 6 Teilen Vinylpyrrolidon und 4 Teilen Vinylacetat (Kollidon^{®}VA 64).

Der Gehalt an Kristallisationsinhibitor im erfindungsgemäßen Transdermalsystem beträgt 0,1 bis 40%, vorzugsweise 2 bis 20%.

Das erfindungsgemäße System weist vorzugsweise einen zusätzlichen Gehalt an wenigstens einem Penetrationsverstärker auf.

Als Penetrationsverstärker können eingesetzt werden:
ein- oder mehrwertige Alkohole wie Ethanol, 1,2-Propandiol oder Benzylalkohol; gesättigte oder ungesättigte Fettalkohole mit 8 bis 18 Kohlenstoffatomen wie Laurylalkohol oder Cetylalkohol; Kohlenwasserstoffe wie Mineralöl; gesättigte und ungesättigte Fettsäuren mit 8 bis 18 Kohlenstoffatomen wie Stearinsäure oder Ölsäure; Fettsäureester mit bis zu 24 Kohlenstoffatomen oder Dicarbonsäurediester mit bis zu 24 Kohlenstoffatomen wie die Methylester, Ethylester, Isopropylester, Butylester, sec.-Butylester, Isobutylester, tert.-Butylester oder Monoglycerinsäureester der Essigsäure, Capronsäure, Laurinsäure, Myristinsäure, Stearinsäure und Palmitinsäure, Phosphatidderivate, wie Lecithin, Terpene, Harnstoff und seine Derivate oder Ether wie Dimethylisosorbid und Diethylenglycolmonoethylether.

Besonders bevorzugte sind Laurylalkohol, 1,2-Propandiol, die Methylester und insbesondere die Isopropylester der Myristinsäure oder Ölsäure, Diisopropyladipat und Diisopropylsebacat, Laurinsäure und Ölsäure, sowie deren Gemische.

Weiterhin können auch Mischungen aus einem oder mehreren penetrationsverstärkenden Mitteln für die erfindungsgemäße transdermal wirksame Formulierung verwendet werden. Hier werden Mischungen von bis zu vier Penetrationsverstärkern eingesetzt. Bevorzugt ist der Einsatz von binären und ternären Penetrationsverstärkergemischen. Am bevorzugtesten ist der Einsatz von binären Penetrationsverstärkergemischen aus hydrophilen mit lipophilen Penetrationsverstärkern eingesetzt.
Beispiele sind Enhancermischungen von Fettsäureestern oder Fettsäuren mit kurzkettigen, ein- oder zweiwertigen Alkoholen im Verhältnis 1:10 bis 10:1. Bevorzugtes Mischungsverhältnis ist 3:1 bis 1:3.

Der Gehalt an Penetrationsverstärker oder Penetrationsverstärkergemisch im erfindungsgemäßen Transdermalsystem beträgt 0,5 bis 40%, vorzugsweise 5 bis 25 %.

Der Matrix können darüber hinaus auch Stabilisatoren wie Cyclodextrine, vorzugsweise das β-Cyclodextrin und seine Derivate, zugesetzt werden.

Weiterhin ist die Erfindung bevorzugt gekennzeichnet durch einen zusätzlichen Gehalt an wenigstens einem Estrogen.
Als Estrogen können eingesetzt werden: Estradiol, Estriol, Ethinylestradiol, deren Derivate wie beispielsweise Mono- und Di-ester wie das Estradiol-3,17β-diproprionat.

Überraschenderweise wird mit (21S)-21-Hydroxy-21-methyl-14,17-ethano-19-norpregna-4,9,15-trien-3,20-dion (Hydroxytriendion) erstmals ein hoch potentes Gestagen zur Verfügung gestellt, das in Matrix-Transdermalsystemen auf Polyacrylatkleber-Basis eine überraschend hohe Löslichkeit von bis zu etwa 20 Gew.-% besitzt. Mittels solcher hochbeladener Systeme können außerordentlich hohe transdermale Flüsse erzielt werden.
Die Kombination von hoher gestagener Potenz, exzellenter Matrix-Beladbarkeit und Hautpenetration des Wirkstoffes ermöglicht es, unter Einsatz von Hydroxytriendion leistungsfähige Hormonersatz- oder Fertilitätskontroll-Transdermalsysteme zur Verfügung zu stellen.

Selbstverständlich ist es möglich, in die Matrix-Transdermalsysteme weitere Hilfsstoffe einzubeziehen, wie die oben erwähnten Kristallisationsinhibitoren und Penetrationsverstärker oder Lösungsvermittler und/oder Lösungsmittel.
Darüber hinaus können Penetrationsverstärker zur Erhöhung der Hautflüsse auch vor Applikation des Transdermalsystems auf die entsprechenden Hautpartien aufgetragen werden.

### Beispiele

### Beispiel 1: Herstellung von Transdermalsystemen mit Hydroxytriendion

Hydroxytriendion wird in ein Becherglas eingewogen und mit 25 g einer 30 %igen Lösung von Kollidon^{®} VA 64 in 2-Propanol. Es werden 10 ml 2-Propanol zugesetzt. Die erhaltene Mischung wird 5 min. gerührt. Die Mischung wird in die vorgelegte Kleberlösung (Gelva^{®} 7881) eingetragen. Die untenstehende Tabelle gibt die im vorgelegten Volumen der Kleberlösung enthaltene Klebertrockenmasse an. Anschließend wird weitere 0,5 h gerührt. Nach polarisationsmikroskopischer Prüfung auf Kristallfreiheit wird die erhaltene Mischung mittels einer 300 µm Rakel auf einen Liner aus fluorpolymerbeschichtetes Polyester (Scotchpak^{®} 1022 Release Liner) gecoatet. Es wird 20 min. bei 70°C getrocknet und anschließend mit einem Backing aus einem Co-Laminat von PVC-PVDC mit Polyester (Saran-Hytrel^{®} Backing) laminiert. Aus dem erhaltenen dreischichtigen Laminat werden runde Pflaster von 10 cm² Fläche gestanzt und in Siegelrandbeutel aus Aluminiumverbundfolie eingeschweißt.

| Formulierung | Gelva^{®} 7881 | Hydroxytriendion | | Kollidon^{®} VA 64 | |
|---|---|---|---|---|---|
| | | Masse | Konz. im TDS (m/m%) | Masse | Konz. im TDS (m/m%) |
| 1 | 42 g | 0,5 g | 1 | 7,5 g | 15 |
| 2 | 41 g | 1,5g | 3 | 7,5 g | 15 |
| 3 | 40 g | 2,5 g | 5 | 7,5 g | 15 |
| 4 | 37,5 g | 5,0 g | 10 | 7,5 g | 15 |
| 5 | 35 g | 7,5 g | 15 | 7,5 g | 15 |

| | | | | | |
|---|---|---|---|---|---|
| Gelva^{®} 7881 ist eine 50 %ige Polyacrylatkleber-Lösung in Ethylacetat (Hersteller: Fa. Solutia). In obiger Tabelle sind die Trockengewichte als Einwaage angegeben. Scotchpak^{®} 1022 Release Liner ist ein Produkt der Fa. 3M, St. Paul, MN, USA. Saran-Hytrel^{®} Backing ist ein Produkt der Fa. Bertek, St. Albans, VT, USA. | | | | | |

### Beispiel 2: Penetrationsstudien

Die Ergebnisse der Penetrationsstudien mit den entsprechenden fünf Formulierungen mit unterschiedlichen Gehalten an Hydroxytriendion sind in den folgenden Tabellen dargestellt.
Die Messungen wurden mit folgendem in-vitro-Diffusionstest durchgeführt:
Eine temperierte Franz-Durchflusszelle wird durch ein 2 cm² großes Stück excidierte Haut von haarlosen Mäusen in ein Donor- und ein Akzeptorkompartiment geteilt, wobei das Stratum corneum der Donorseite zugewandt ist. Eine 3%ige oder 5 %ige Lösung von Hydroxypropyl-β-cyclodextrin in Puffer wird mit Hilfe einer pneumatischen Pumpe aus einem temperierten Vorratsbehälter durch das Akzeptorkompartiment gepumpt und mit Hilfe eines Fraktionssammlers in Glasvials gesammelt, die in bestimmten Zeitabständen ausgetauscht werden.
Auf der Donorseite werden die wirkstoffhaltigen Pflaster aufgeklebt.
Der Gehalt an Wirkstoffen wird in den einzelnen Fraktionen mittels HPLC/UV bzw. GC/FID bestimmt.
Der transdermale in-vitro-Hautfluss wird in ng/cm²/h angegeben:

**Formulierung 1: 1 % Hydroxytriendion**

| [h] | 1 | 2 | 3 | 4 | MW | SD | CV |
|---|---|---|---|---|---|---|---|
| 1 | 2 | 1 | 0 | 0 | 1 | 1 | 86,6 % |
| 3 | 16 | 13 | 2 | 6 | 9 | 6 | 70,4 % |
| 5 | 55 | 36 | 11 | 27 | 32 | 18 | 57,2 % |
| 10 | 110 | 57 | 45 | 72 | 71 | 28 | 39,8 % |
| 18 | 79 | 59 | 51 | 76 | 66 | 13 | 20,2 % |
| 26 | 77 | 44 | 48 | 111 | 70 | 31 | 44,3 % |
| 34 | 56 | 32 | 47 | 52 | 47 | 11 | 22,6 % |
| 42 | 62 | 26 | 40 | 51 | 45 | 15 | 33,9 % |
| 50 | 40 | 20 | 39 | 42 | 35 | 10 | 29,0 % |

**Formulierung 2: 3 % Hydroxytriendion**

| [h] | 1 | 2 | 3 | 4 | MW | SD | CV |
|---|---|---|---|---|---|---|---|
| 1 | 1 | 1 | 0 | 1 | 1 | 0 | 71,1 % |
| 3 | 20 | 24 | 4 | 16 | 16 | 9 | 54,3 % |
| 5 | 64 | 111 | 17 | 61 | 63 | 38 | 60,6 % |
| 10 | 118 | 171 | 109 | 207 | 151 | 46 | 30,6 % |
| 18 | 118 | 144 | 166 | 161 | 148 | 22 | 14,6 % |
| 26 | 106 | 147 | 197 | 158 | 152 | 37 | 24,5 % |
| 34 | 71 | 115 | 160 | 142 | 122 | 39 | 31,8 % |
| 42 | 54 | 114 | 120 | 116 | 101 | 31 | 30,9 % |
| 50 | 46 | 91 | 68 | 103 | 77 | 25 | 32,8 % |

**Formulierung 3: 5 % Hydroxytriendion**

| [h] | 1 | 2 | 3 | 4 | MW | SD | CV |
|---|---|---|---|---|---|---|---|
| 1 | 2 | 1 | 1 | 2 | 1 | 1 | 67,3 % |
| 3 | 33 | 18 | 13 | 24 | 22 | 8 | 38,3 % |
| 5 | 104 | 57 | 48 | 86 | 74 | 26 | 34,9 % |
| 10 | 177 | 212 | 302 | 251 | 236 | 54 | 22,8 % |
| 18 | 168 | 280 | 228 | 345 | 255 | 75 | 29,4 % |
| 26 | 168 | 393 | 231 | 393 | 296 | 115 | 38,7 % |
| 34 | 196 | 206 | 240 | 263 | 226 | 31 | 13,7 % |
| 42 | 152 | 162 | 182 | 180 | 169 | 15 | 8,7 % |
| 50 | 147 | 147 | 221 | 138 | 163 | 39 | 23,9 % |

**Formulierung 4: 10 % Hydroxytriendion**

| [h] | 1 | 2 | 3 | 4 | MW | SD | CV |
|---|---|---|---|---|---|---|---|
| 1 | 0 | 5 | 3 | 1 | 2 | 2 | 87,9 % |
| 3 | 16 | 74 | 60 | 35 | 46 | 26 | 55,6 % |
| 5 | 71 | 239 | 192 | 123 | 156 | 74 | 47,6 % |
| 10 | 349 | 466 | 428 | 477 | 430 | 58 | 13,5 % |
| 18 | 451 | 262 | 494 | 291 | 375 | 115 | 30,8 % |
| 26 | 695 | 269 | 463 | 242 | 417 | 209 | 50,2 % |
| 34 | 387 | 272 | 294 | 209 | 290 | 74 | 25,5 % |
| 42 | 257 | 232 | 242 | 165 | 224 | 41 | 18,2 % |
| 50 | 321 | 102 | 145 | 147 | 179 | 97 | 54,3 % |

**Formulierung 5: 15 % Hydroxytriendion**

| h | 1 | 2 | 4 | MW | SD | CV |
|---|---|---|---|---|---|---|
| 1 | 2 | 5 | 5 | 4 | 2 | 49,7 % |
| 3 | 43 | 69 | 68 | 60 | 14 | 23,8 % |
| 5 | 213 | 182 | 187 | 194 | 17 | 8,6 % |
| 10 | 486 | 408 | 543 | 479 | 67 | 14,1 % |
| 18 | 374 | 404 | 276 | 352 | 67 | 19,0 % |
| 26 | 400 | 487 | 263 | 383 | 113 | 29,0 % |
| 34 | 386 | 218 | 220 | 275 | 96 | 35,1 % |
| 42 | 261 | 184 | 174 | 206 | 48 | 23,1% |
| 50 | 271 | 176 | 160 | 202 | 60 | 29,6 % |

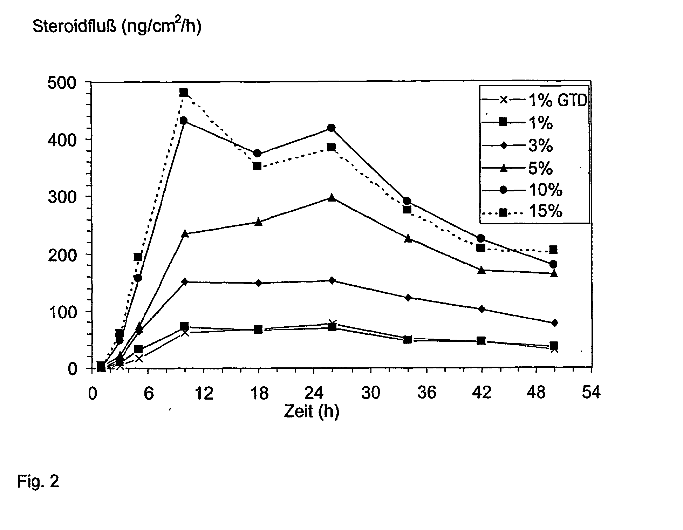

Die Mittelwerte und Standardabweichungen für die transdermalen in-vitro-Hautflüsse ergeben sich aus der nachfolgenden Tabelle II und sind in Fig. 2 graphisch dargestellt

**Tabelle II**

| Mittelwerte und Standardabweichungen: | | | | | |
|---|---|---|---|---|---|
| [h] | 1 % | 3% | 5% | 10% | 15% |
| 1 | 1±1 | 1±0 | 1±1 | 2±2 | 4±2 |
| 3 | 9±6 | 16±9 | 22±8 | 46±26 | 60±14 |
| 5 | 32±18 | 63±38 | 74±26 | 156±74 | 194±17 |
| 10 | 71±28 | 151±46 | 236±54 | 430±58 | 479±67 |
| 18 | 66±13 | 148±22 | 255±75 | 375±115 | 352±67 |
| 26 | 70±31 | 152±37 | 296±115 | 417±209 | 383±113 |
| 34 | 47±11 | 122±39 | 226±31 | 290±74 | 275±96 |
| 42 | 45±15 | 101±31 | 169±15 | 224±41 | 206±48 |
| 50 | 35±10 | 77±25 | 163±39 | 179±97 | 202±60 |

### Beispiel 3: Vergleichsversuch zwischen Gestoden und Hydroxytriendion

Zum Vergleich wurden dieselben Messungen der transdermalen in-vitro-Hautflüsse - bei ansonsten gleicher Formulierung - mit Gestoden (GTD) durchgeführt. Die entsprechenden Ergebnisse ergeben sich aus den nachfolgenden Tabellen.

**Transdermale in-vitro-Hautfluss in ng/cm²/h (1 % Gew.-% Gestoden)**

| [h] | 1 | 2 | 3 | 4 | MW | SD | CV |
|---|---|---|---|---|---|---|---|
| 1 | 0 | 0 | 0 | 0 | 0 | 0 | - |
| 3 | 1 | 4 | 2 | 5 | 3 | 2 | 64,3 % |
| 5 | 5 | 31 | 14 | 18 | 17 | 11 | 64,3 % |
| 10 | 39 | 72 | 80 | 58 | 62 | 18 | 28,6 % |
| 18 | 77 | 46 | 110 | 36 | 67 | 34 | 49,8 % |
| 26 | 120 | 45 | 111 | 33 | 77 | 45 | 57,8 % |
| 34 | 50 | 39 | 83 | 31 | 51 | 23 | 44,6 % |
| 42 | 47 | 33 | 74 | 25 | 45 | 22 | 49,0 % |
| 50 | 37 | 27 | 44 | 22 | 32 | 10 | 30,0 % |

Ein Matrix-TDS, in dem der Wirkstoff in gelöster Form vorliegt, lässt sich gemäß Beispiel 1 mit Gestoden lediglich bis ca. 1 % (m/m) herstellen. Darüber hinaus kommt es zu Rekristallisationserscheinungen des Gestodens. Der Hautfluss lässt sich nicht entscheidend steigern.
Zwar sind die Hautflüsse für 1%-Matrix-TDS zwischen Hydroxytriendion und Gestoden vergleichbar, doch lassen sich die Hautflüsse von Hydroxytriendion deutlich steigern dadurch dass, Hydroxytriendion in den erfindungsgemäßen Matrix-TDS bis ca. 20 % (m/m) gelöst beladbar ist.

## Patentansprüche

1. Matrix-Transdermalsystem enthaltend (21S)-21-Hydroxy-21-methyl-14,17-ethano-19-norpregna-4,9,15-trien-3,20-dion **dadurch gekennzeichnet, dass** die Matrix Polyacrylatkleber umfasst.

2. Transdermalsystem nach Anspruch 1, **dadurch gekennzeichnet, dass** der Polyacrylatkleber ein Copolymer von zumindest 2 der folgenden Monomere ist: 2-Ethylhexylacrylat, Hydroxyethylhexylacrylat, Vinylacetat, Vinylpyrrolidon.

3. Transdermalsystem nach Anspruch 2, **dadurch gekennzeichnet, dass** der Polyacrylatkleber ein Copolymer aus 2-Ethylhexylacrylat und Hydroxyethylacrylat oder ein Copolymer dieser Monomere mit Vinylacetat und 2-Ethyl-hexylacrylat-N-vinyl-2-pyrrolidon ist.

4. Transdermalsystem nach einem der Ansprüche 1 bis 3, **gekennzeichnet durch** einen Gehalt von 0,1 bis 20 Gew.-%, vorzugsweise 1 bis 15% (21 S)-21-Hydroxy-21-methyl-14,17-ethano-19-norpregna-4,9,15-trien-3,20-dion in der Matrix.

5. Transdermalsystem nach einem der Ansprüche 1 bis 4, **dadurch gekennzeichnet, dass** die Matrix wenigstens einen Kristallisationsinhibitor umfasst.

6. Transdermalsystem nach Anspruch 5, **dadurch gekennzeichnet, dass** die Matrix wenigstens ein N-Vinyllactam-Polymer wie N-Vinyl-1-Azacycloheptan-2-on-Homopolymer, N-Vinyl-piperidin-2-on-Homopolymer, Polymere des Vinylpyrrolidons wie Polyvidon, oder Co-Polymere des Vinylpyrrolidons mit Vinylacetat umfasst.

7. Transdermalsystem nach einem der Ansprüche 1 bis 6, **gekennzeichnet durch** einen zusätzlichen Gehalt an wenigstens einem der folgenden Penetrationsverstärker:
ein- oder mehrwertige Alkohole wie Ethanol, 1,2-Propandiol oder Benzylalkohol; gesättigte oder ungesättigte Fettalkohole mit 8 bis 18 Kohlenstoffatomen wie Laurylalkohol oder Cetylalkohol; Kohlenwasserstoffe wie Mineralöl; gesättigte und ungesättigte Fettsäuren mit 8 bis 18 Kohlenstoffatomen wie Stearinsäure oder Ölsäure; Fettsäureester mit bis zu 24 Kohlenstoffatomen oder Dicarbonsäurediester mit bis zu 24 Kohlenstoffatomen wie die Methylester, Ethylester, Isopropylester, Butylester, sek.-Butylester, Isobutylester, tert.-Butylester oder Monoglycerinsäureester der Essigsäure, Capronsäure, Laurinsäure, Myristinsäure, Stearinsäure und Palmitinsäure, Phosphatidderivate, wie Lecithin, Terpene, Harnstoff und seine Derivate oder Ether wie Dimethylisosorbid und Diethylenglycolmonoethylether.

8. Transdermalsystem nach Anspruch 7, **gekennzeichnet durch** einen Gehalt an wenigstens einem der folgenden Penetrationsverstärker: Laurylalkohol, 1,2-Propandiol, die Methylester und insbesondere die Isopropylester der Myristinsäure oder Ölsäure, Düsopropyladipat und Diisopropylsebacat, Laurinsäure und Ölsäure, sowie deren Gemische.

9. Transdermalsystem nach einem der vorangehenden Ansprüche, **gekennzeichnet durch** einen zusätzlichen Gehalt an wenigstens einem Estrogen wie Estradiol, Estriol, Ethinylestradiol, deren Derivate wie beispielsweise Mono- und Di-ester wie Estradiol-3,17β-diproprionat.

10. Transdermalsystem nach einem der vorangehenden Ansprüche, **gekennzeichnet durch** einen zusätzlichen Gehalt an wenigstens einem Stabilisator wie beispielsweise Cyclodextrin.

## Claims

1. Matrix-transdermal system that contains (21S)-21-hydroxy-21-methyl-14,17-ethano-19-norpregna-4,9,15-triene-3,20-dione, **characterized in that** the matrix comprises polyacrylate adhesive.

2. Transdermal system according to Claim 1, charcterized in that the polyacrylate adhesive is a copolymer of at least two of the following monomers: 2-ethylhexylacrylate, hydroxyethylhexylacrylate, vinyl acetate, vinyl pyrrolidone.

3. Transdermal system according to Claim 2, **characterized in that** the polyacrylate adhesive is a copolymer that consists of 2-ethylhexylacrylate and hydroxyethylacrylate or a copolymer of these monomers with vinyl acetate and 2-ethylhexylacrylate-N-vinyl-2-pyrrolidone.

4. Transdermal system according to one of Claims 1 to 3, **characterized by** a content of 0.1 to 20% by weight, preferably 1 to 15% of (21S)-21-hydroxy-21-methyl-14,17-ethano-19-norpregna-4,9,15-triene-3,20-dione in the matrix.

5. Transdermal system according to one of Claims 1 to 4, **characterized in that** the matrix comprises at least one crystallization inhibitor.

6. Transdermal system according to Claim 5, **characterized in that** the matrix comprises at least one N-vinyllactam polymer such as N-vinyl-1-azacycloheptan-2-one homopolymer, N-vinyl-piperidin-2-one homopolymer, polymers of vinylpyrrolidone such as polyvidone or co-polymers of vinylpyrrolidone with vinyl acetate.

7. Transdermal system according to one of Claims 1 to 6, **characterized by** an additional content of at least one of the following penetration intensifiers:
monovalent or multivalent alcohols such as ethanol, 1,2-propanediol or benzyl alcohol; saturated or unsaturated fatty alcohols with 8 to 18 carbon atoms, such as lauryl alcohol or cetyl alcohol; hydrocarbons such as mineral oil; saturated and unsaturated fatty acids with 8 to 18 carbon atoms such as stearic acid or oleic acid; fatty acid esters with up to 24 carbon atoms or dicarboxylic acid diesters with up to 24 carbon atoms, such as the methyl esters, ethyl esters, isopropyl esters, butyl esters, sec-butyl esters, isobutyl esters, tert-butyl esters or monoglyceric acid esters of acetic acid, caproic acid, lauric acid, myristic acid, stearic acid and palmitic acid, phosphatide derivatives, such as lecithin, terpenes, urea and its derivatives or ethers, such as dimethyl isosorbide and diethylene glycol monoethyl ether.

8. Transdermal system according to Claim 7, **characterized by** a content of at least one of the following penetration intensifiers: lauryl alcohol, 1,2-propanediol, the methyl esters and especially the isopropyl esters of myristic acid or oleic acid, diisopropyl adipate and diisopropyl sebacate, lauric acid and oleic acid, as well as mixtures thereof.

9. Transdermal system according to one of the preceding claims, **characterized by** an additional content of at least one estrogen such as estradiol, estriol, ethinylestradiol, derivatives thereof such as, for example, monoesters and diesters such as estradiol-3,17β-diproprionate.

10. Transdermal system according to one of the preceding claims, **characterized by** an additional content of at least one stabilizer, such as, for example, cyclodextrin.

## Revendications

1. Système transdermique à matrice, contenant de la (21S)-21-hydroxy-21-méthyl-14,17-éthano-19-norpregna-4,9,15-triène-3,20-dione, **caractérisé en ce que** la matrice contient un adhésif à base de polyacrylate.

2. Système transdermique selon la revendication 1, **caractérisé en ce que** l'adhésif à base de polyacrylate est un copolymère d'au moins 2 des monomères suivants : acrylate de 2-éthylhexyle, acrylate d'hydroxyéthylhexyle, acétate de vinyle, vinylpyrrolidone.

3. Système transdermique selon la revendication 2, **caractérisé en ce que** l'adhésif à base de polyacrylate est un polymère d'acrylate de 2-éthylhexyle et d'acrylate d'hydroxyéthyle ou un copolymère de ces monomères avec de l'acétate de vinyle et du (acrylate de 2-éthylhexyle)-N-vinyl-2-pyrrolidone.

4. Système transdermique selon l'une quelconque des revendications 1 à 3, **caractérisé par** une teneur de 0,1 à 20% en poids, de préférence de 1 à 15% en (21S)-21-hydroxy-21-méthyl-14,17-éthano-19-norpregna-4,9,15-triène-3,20-dione dans la matrice.

5. Système transdermique selon l'une quelconque des revendications 1 à 4, **caractérisé en ce que** la matrice contient au moins un inhibiteur de cristallisation.

6. Système transdermique selon la revendication 5, **caractérisé en ce que** la matrice contient au moins un polymère de N-vinyllactame, tel qu'un homopolymère de N-vinyl-1-azacycloheptan-2-one, un homopolymère de N-vinyl-pipéridine-2-one, des polymères de la vinylpyrrolidone telle que la polyvidone, ou des copolymères de la vinylpyrrolidone avec de l'acétate de vinyle.

7. Système transdermique selon l'une quelconque des revendications 1 à 6, **caractérisé par** une teneur supplémentaire en un moins un des agents de renforcement de la pénétration suivants : les alcools monovalents ou polyvalents, tels que l'éthanol, le 1,2-propandiol ou l'alcool benzylique ; les alcools gras saturés ou insaturés, comprenant 8 à 18 atomes de carbone, tels que l'alcool laurylique ou cétylique ; les hydrocarbures tels que l'huile minérale ; les acides gras saturés et insaturés, comprenant 8 à 18 atomes de carbone, tels que l'acide stéarique ou l'acide oléique ; les esters d'acide gras comprenant jusqu'à 24 atomes de carbone ou les diesters d'acide dicarboxylique comprenant jusqu'à 24 atomes de carbone, tels que les esters de méthyle, d'éthyle, d'isopropyle, de butyle, de sec-butyle, d'isobutyle, de tert-butyle ou les esters de l'acide monoglycérique de l'acide acétique, caproïque, laurique, myristique, stéarique et palmitique, les dérivés du phosphate, tels que la lécithine, les terpènes, l'urée et ses dérivés ou les éthers tels que le diméthylisosorbide et le diéthylèneglycolmonoéthyléther.

8. Système transdermique selon la revendication 7, **caractérisé par** une teneur supplémentaire en un moins un des agents de renforcement de la pénétration suivants : l'alcool laurylique, le 1,2-propanediol, l'ester méthylique et en particulier l'ester isopropylique de l'acide myristique ou oléique, l'adipate de diisopropyle et le sébacate de diisopropyle, l'acide laurique et l'acide oléique, ainsi que leurs mélanges.

9. Système transdermique selon l'une quelconque des revendications précédentes, **caractérisé par** une teneur supplémentaire en au moins un estrogène, tel que l'estradiol, l'estriol, l'éthynylestradiol, leurs dérivés tels que par exemple les monoesters et les diesters, tels que le 3,17β-diproprionate d'estradiol.

10. Système transdermique selon l'une quelconque des revendications précédentes, **caractérisé par** une teneur supplémentaire en au moins un stabilisateur tel que par exemple la cyclodextrine.
